# FASCICULE DE BREVET EUROPEEN

(11) **EP 2 810 686 B1**
(45) Date de publication et mention de la délivrance du brevet: **23.03.2016**
(21) Numéro de dépôt: 14165462.4
(22) Date de dépôt: 22.04.2014
(51) Int. Cl.: A61N 1/05, A61N 1/362, A61N 1/372, A61M 25/00

(54) **Ensemble implantable dans le réseau veineux coronarien pour la stimulation d'une cavité cardiaque gauche**
Einheit zum Implantieren in den koronaren Venenkreislauf zur Stimulation einer linken Herzhöhle
Assembly suitable for implantation in the coronary venous network for stimulation of a left heart chamber

(30) Priorité: 04.06.2013 FR 1355126
(43) Date de publication de la demande: 10.12.2014
(73) Titulaire: Sorin CRM SAS, 92140 Clamart Cedex (FR)
(72) Inventeur: Régnier, Willy, 91160 Longjumeau (FR); Shan, Nicolas, 91260 Juvisy sur Orge (FR); Ollivier, Jean-François, 91190 Villiers Le Bacle (FR)
(74) Mandataire: Dupuis-Latour, Dominique

(56) Documents cités:
- EP-A1- 2 275 170
- EP-A1- 2 455 131
- EP-A1- 2 559 453
- US-A1- 2006 247 751

## Description

L'invention concerne les "dispositifs médicaux implantables actifs" tels que définis par la Directive 90/385/CEE du 20 juin 1990 du Conseil des communautés européennes, notamment les implants permettant de surveiller en continu le rythme cardiaque et délivrer si nécessaire au coeur des impulsions électriques de stimulation ou de resynchronisation.

Elle concerne plus précisément les sondes de stimulation cardiaque destinées à être implantées dans le réseau coronarien du coeur pour permettre la stimulation d'une cavité gauche, ventricule ou oreillette.

À la différence des cavités droites, lorsque l'on veut stimuler une cavité gauche, on choisit le plus souvent d'introduire une sonde non pas dans la cavité à stimuler mais dans le réseau coronarien, la sonde étant pourvue d'une électrode qu'il conviendra d'appliquer contre la paroi de l'épicarde et d'orienter vers le ventricule gauche ou l'oreillette gauche, selon le cas.

Une sonde de ce type est par exemple le modèle *Situs LV,* commercialisé par Sorin CRM (Clamart, France) et décrit dans le EP 0 993 840 A1 (ELA Medical).

L'introduction d'une telle sonde se fait par le sinus coronaire débouchant dans l'oreillette droite, donc par voie endocavitaire. La sonde est ensuite orientée et poussée le long du réseau des veines coronaires jusqu'au site de stimulation choisi. Cette intervention est très délicate compte tenu des particularités du réseau veineux et de ses voies d'accès, avec notamment le passage de valvules et tortuosités ainsi que la diminution progressive de diamètre du conduit au fur et à mesure de la progression de la sonde dans la veine coronaire sélectionnée.

Une fois la veine cible atteinte, il faut trouver un site de stimulation satisfaisant, et s'assurer que le point de stimulation choisi ne génère pas de stimulation phrénique.

Par ailleurs, une tendance des développements récents en matière de sondes de stimulation du ventricule gauche est la réduction du diamètre de la partie implantable dans le réseau coronaire. La taille du corps de sonde est en effet un facteur directement lié aux capacités de guidage contrôlé de la sonde dans le réseau coronaire veineux, pour pouvoir y sélectionner des sites de stimulation particuliers situés dans certaines veines collatérales.

Ainsi, le EP 2 581 107 A1 (Sorin CRM SAS) décrit une sonde constituée dans sa partie active, distale, par un microcâble présentant un diamètre de l'ordre de 0,5 à 2 French (0,17 à 0,66 mm). Ce microcâble comprend un câble de coeur électriquement conducteur formé par un ou plusieurs torons d'une pluralité de brins composites, avec une couche d'isolement en polymère entourant partiellement le câble de coeur. La couche d'isolement est dénudée ponctuellement de façon à mettre à nu le microcâble en un ou plusieurs points constituant ensemble un réseau d'électrodes reliées en série, l'extrémité libre du toron étant par ailleurs dotée d'une électrode distale rapportée.

Le EP 2 455 131 A1 (Sorin CRM SAS) décrit une sonde du même type, dans laquelle le microcâble coulisse dans une lumière d'un corps de sonde, d'où il peut émerger sur une longueur de 1 à 200 mm au-delà du débouché de ce corps de sonde. L'extrémité distale du corps de sonde est pourvue d'un manchon en silicone assurant sa retenue dans une région médiane du vaisseau cible. À partir de cette position, le microcâble est déployé dans le vaisseau jusqu'à ce que sa partie active distale (portant les parties dénudées formant le réseau d'électrodes) atteigne la région cible située dans une zone profonde du réseau coronarien.

Dans un autre mode de réalisation décrit par ce document, la sonde comprend une pluralité de microcâbles logés dans autant de lumières respectives distinctes d'un même corps de sonde. Les débouchés des différentes lumières sont décalés axialement sur le corps de sonde, qui présente de ce fait une pluralité d'ouvertures latérales d'où émergent successivement les parties actives de chacun des microcâbles.

Le très faible diamètre du microcâble permet d'exploiter toute la longueur de la veine et de canuler des veines de très faible diamètre, non exploitées à ce jour du fait de la taille excessive des sondes coronaires conventionnelles. Il devient de ce fait possible de traiter de nouvelles régions difficilement atteignables et ainsi d'utiliser de façon optimale toutes les veines présentes dans la zone basale, en particulier pour éviter le risque de stimulation phrénique qui augmente généralement lorsque la sonde est trop distale.

Par ailleurs, la multiplication des points de stimulation dans une zone profonde du réseau coronarien permet (contrairement aux sondes traditionnelles) de stimuler simultanément plusieurs zones de l'épicarde dans la région de stimulation, en améliorant de ce fait les chances d'une resynchronisation optimale du myocarde.

Avec une telle microsonde, il est même possible de traverser des anastomoses (passages présents à l'extrémité de certaines veines vers une autre veine) avec possibilité de faire progresser la microsonde dans une première veine (veine "aller") puis par l'anastomose vers une deuxième veine (veine "retour") en remontant celle-ci, pour permettre en particulier une stimulation du ventricule gauche à partir de deux régions distinctes et distantes.

Enfin, la structure de cette microsonde lui procure une grande robustesse qui en garantit la biostabilité à long terme.

L'une des difficultés rencontrées avec ce type de microsonde réside dans l'évaluation électrique du site de stimulation avant le placement définitif de la microsonde.

En effet, l'implantation d'une microsonde du type précité repose sur l'utilisation d'un cathéter très fin introduit jusqu'à la veine-cible par une technique conventionnelle, avec introduction d'un fil-guide dans le réseau veineux, puis enfilage du cathéter sur ce fil-guide et enfin retrait du fil-guide. Le cathéter mis en place permet de guider la microsonde jusqu'au site de stimulation, puis ce cathéter est retiré afin de découvrir les électrodes de la microsonde et rendre ainsi celles-ci fonctionnelles.

Ce mode opératoire ne permet pas d'anticiper la qualité des performances de stimulation avant de découvrir les électrodes de la microsonde, dans la position finale de celle-ci.

Par ailleurs, ce type de sonde constituée d'un microcâble n'a pas de très bonnes performances de *tracking,* c'est-à-dire d'aptitude à progresser dans le réseau veineux par manipulation de son extrémité proximale en poussée et en torsion.

Un microcâble est en effet beaucoup plus déformable qu'un fil-guide, qui est spécialement conçu pour naviguer dans le réseau veineux et possède les propriétés requises de "torquabilité" (aptitude à transmettre sur toute sa longueur, jusqu'à l'extrémité distale, un mouvement de rotation imprimé par une poignée de manoeuvre depuis l'extrémité proximale) et de "pus-habilité" (aptitude à progresser dans le réseau biologique sans arc-boutement, sous l'effet d'une poussée exercée depuis l'extrémité proximale avec la poignée de manoeuvre), propriétés nécessaires à la navigation dans le réseau coronarien.

Il n'est de ce fait pas envisageable d'introduire et de guider directement un microcâble dans le réseau veineux. Pour ces mêmes raisons, il est également très difficile d'en modifier la position une fois qu'il a été mis en place, notamment pour rechercher si nécessaire un meilleur site de stimulation, et en tout cas impossible de le reculer pour aller ensuite sélectionner une autre veine ou passer une anastomose.

Le problème à résoudre est de pouvoir évaluer le plus tôt possible dans la procédure d'implantation la meilleure position de placement de la microsonde et de ses électrodes, afin d'être en mesure si nécessaire de modifier cette position ou même d'envisager un repositionnement de la microsonde dans une autre veine du réseau coronaire.

L'idée de base de l'invention consiste à combiner le microcâble à un cathéter implantable à demeure (qui de ce fait sera différent des cathéters conventionnels, qui ne sont pas destinés à demeurer en place au-delà de la manoeuvre de pose de la sonde). Ce cathéter implantable, ci-après désigné "microcathéter", joue le rôle d'isolant pour le microcâble, et il est pourvu d'une ou plusieurs fenêtres ménagées dans la paroi du microcathéter, ces fenêtres autorisant localement la transmission d'un champ électrique de stimulation et définissant de cette manière les zones de stimulation de la microsonde (la "microsonde" étant entendue comme la combinaison du microcâble et du microcathéter).

Microcathéter et microcâble sont séparables, car ils sont destinés à être implantés successivement et non simultanément comme dans le cas d'une microsonde à microcâble pourvue d'une couche d'isolation.

Au surplus, la mise en place du microcâble dans le microcathéter laisse subsister entre eux une possibilité de déplacement axial relatif (fonction télescopique) de manière à pouvoir déplacer les fenêtres, et donc les zones de stimulation, une fois l'ensemble en place dans la veine.

Par ailleurs, selon un aspect particulier de l'invention, l'évaluation électrique des zones de stimulation pourra être effectuée juste après la pose du microcathéter, et avant l'introduction du microcâble, par l'intermédiaire du fil-guide ayant servi à poser le microcathéter, ce fil-guide étant laissé en place pendant le temps de cette évaluation.

Dans cette configuration, c'est le fil-guide (en matériau métallique) qui jouera le rôle de conducteur électrique positionné en vis-à-vis des fenêtres du microcathéter : le médecin pourra alors repositionner les zones de stimulation en déplaçant vers l'avant ou vers l'arrière le microcathéter le long du fil-guide.

Si la voie choisie n'offre pas un seuil de stimulation satisfaisant, le médecin pourra alors reculer le microcathéter pour découvrir l'extrémité du fil-guide, et sélectionner avec ce fil-guide une autre anastomose, puis recommencer rapidement l'évaluation d'une nouvelle veine sans avoir besoin de retirer ni le fil-guide ni le microcathéter.

Il devient ainsi possible d'optimiser le temps d'implantation par une évaluation rapide du site de stimulation, et également d'offrir au médecin davantage de possibilités dans le choix éventuel des anastomoses et dans la position des zones de stimulation, avant même l'introduction du microcathéter.

Une fois que le médecin aura validé la position de stimulation, il n'aura qu'à substituer le microcâble au fil-guide pour terminer la procédure d'implantation. Les fenêtres du microcathéter n'ayant pas changé de place durant cette substitution, la position des sites n'aura pas été modifiée et restera celle qui avait fait l'objet de l'évaluation électrique.

Plus précisément, l'invention propose un ensemble, implantable dans le réseau veineux coronarien, pour la stimulation d'une cavité cardiaque gauche au moyen d'un générateur de dispositif médical actif implantable, notamment un stimulateur ou resynchroniseur cardiaque.

Cet ensemble comprend, en combinaison : un microcâble en matériau conducteur, comprenant en partie proximale des moyens de couplage au générateur du dispositif médical actif implantable ; et un cathéter en matériau isolant, comprenant un tube creux logeant dans une lumière interne le microcâble avec possibilité de translation axiale relative entre cathéter et microcâble.

De façon caractéristique de l'invention, le cathéter est un microcathéter implantable de diamètre au plus égal à 2 French (0,66 mm), réalisé en matériau(x) biocompatible(s) adapté(s) à une implantation permanente dans des réseaux veineux. Dans sa partie distale, il comprend au moins une fenêtre latérale comprenant un orifice traversant ménagé sur la paroi du tube creux, et apte à mettre en regard, sans obstacle galvanique, une région de la surface du microcâble située dans ladite lumière interne au droit de cette fenêtre avec un site de stimulation défini sur la paroi de la veine-cible en regard de la fenêtre du microcathéter, ladite région formant électrode de stimulation. Dans sa partie distale, le microcâble est un microcâble non isolé au moins dans la région de la fenêtre du microcathéter. Par ailleurs, le microcathéter est apte à être déplacé télescopiquement sur le microcâble dans ladite partie distale entre une position déployée, en rapprochement des extrémités distales respectives du microcâble et du microcathéter, et une position rétractée, en éloignement desdites extrémités distales respectives, de manière à modifier la position dudit site de stimulation de la veine-cible par l'effet du déplacement du microcathéter sur le microcâble.

L'orifice traversant de la ou des fenêtre(s) latérale(s) peut être un orifice vide, définissant un volume libre, ou bien être rempli d'un matériau électriquement conducteur. Dans l'un ou l'autre cas, cet orifice autorise localement la transmission du champ électrique de stimulation et définit de cette manière une zone de stimulation de la microsonde.

Selon diverses caractéristiques subsidiaires avantageuses :
- en position déployée, le microcâble n'émerge pas au-delà de l'extrémité distale du tube creux du microcathéter ;
- le microcathéter comprend dans sa région proximale des moyens de solidarisation au microcâble en une position axiale relative, finale, comprise entre la position déployée et la position rétractée ;
- le microcâble comprend au moins un manchon de conformation, gainant le microcâble dans une région de celui-ci située à distance de la fenêtre du microcathéter ;
- la course de déplacement du microcathéter sur se microcâble entre la position déployée et la position rétractée est d'au plus 50 mm ;
- la surface unitaire de la au moins une fenêtre latérale est d'au moins 0,2 mm², et si l'ensemble comprend une pluralité de fenêtres latérales, leur surface cumulée est d'au plus 6 mm² ;
- le microcathéter comprend au moins un groupe d'une pluralité de fenêtres latérales réparties circonférentiellement en une même position axiale du tube creux.

Selon un autre aspect de l'invention, l'ensemble comprend en outre un fil-guide amovible non implantable, ce fil-guide étant apte, après avoir été préalablement introduit dans le réseau veineux coronaire jusqu'à une veine-cible, à recevoir le tube creux du microcathéter enfilé dessus, et à permettre par coulissement le guidage du microcathéter jusqu'à la veine-cible. Le fil-guide est en un matériau conducteur non isolé, et il est muni à sa partie proximale de moyens de couplage à un générateur d'impulsions de stimulation, et le microcathéter est apte à être déplacé télescopiquement sur le fil-guide entre lesdites positions déployée et rétractée, de manière à définir ledit site de stimulation sur la paroi de la veine-cible en regard de la fenêtre du microcathéter, le fil-guide dans la région de la fenêtre formant électrode de stimulation.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 illustre de façon générale le myocarde, avec les principales veines du réseau coronaire, où a été introduite une microsonde selon l'invention pour la stimulation d'une cavité gauche du coeur.
La Figure 2 est une vue d'ensemble de la microsonde de l'invention avec ses deux éléments séparés, microcathéter et microcâble, incluant une vue agrandie de la partie distale de ceux-ci.
La Figure 3 illustre la possibilité de déplacement du microcathéter sur le microcâble, pour l'ajustement local des zones de stimulation.
La Figure 4 illustre également la possibilité d'ajustement présentée Figure 3, avec le microcathéter en place dans la veine coronaire.
La Figure 5 illustre les divers diamètres de l'ensemble microcathéter/microcâble en zone distale de la sonde de l'invention.
La Figure 6a à 6f illustre différentes réalisations possibles des fenêtres ménagées dans le microcathéter de la sonde de l'invention.
La Figure 7 est un organigramme explicitant les étapes successives de la procédure d'implantation de la microsonde selon l'invention.

On va maintenant décrire un exemple de réalisation et de mise en oeuvre de la sonde selon l'invention.

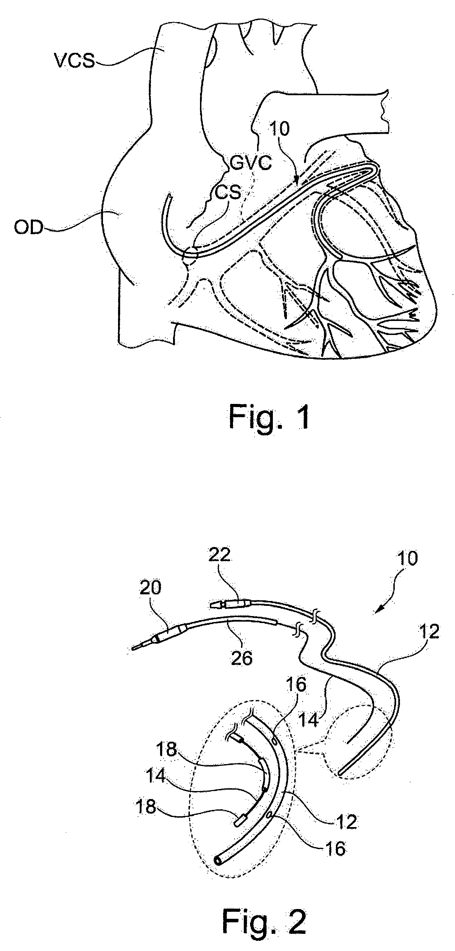

La Figure 1 illustre de façon générale le myocarde et les principaux vaisseaux du réseau coronaire dans lequel on a introduit une microsonde 10 par voie endocavitaire via la veine cave supérieure VCS, l'oreillette droite OD, l'entrée CS du sinus coronaire veineux et la grande veine coronaire GVC. La microsonde 10, qui sera décrite en détail plus bas, présente un diamètre au plus égal à 2 French (0,66 mm), typiquement de l'ordre de 0,5 à 2 French (0,16 à 0,66 mm) et comporte dans sa partie distale une ou plusieurs électrodes de détection/stimulation destinées notamment à stimuler le ventricule gauche à partir d'un ou plusieurs sites correspondants situés dans une veine du réseau coronaire profond, si besoin après traversée d'une anastomose (une anastomose est un passage existant, à l'extrémité de certaines veines du réseau coronaire, vers une autre veine, avec de ce fait possibilité de communication entre deux veines distinctes au niveau de l'anastomose, via leurs extrémités distales respectives) : grâce à une telle configuration, il est possible non seulement de stimuler le ventricule gauche à partir de points situés profondément dans une veine du réseau coronaire, mais également, via l'anastomose, dans des régions proximales de veines dans lesquelles il aurait été difficile de stabiliser ou de fixer des sondes conventionnelles de stimulation du ventricule gauche.

La Figure 2 illustre de façon générale la structure de la microsonde 10 de l'invention, qui est constituée d'un microcathéter 12 avec un tube creux dans la lumière duquel est introduit un microcâble 14, dans les conditions que l'on décrira ci-dessous. Dans sa partie distale (illustrée de façon plus visible dans le détail de la Figure 2), le microcathéter 12 est muni d'une ou plusieurs fenêtres 16 formées par un orifice traversant ménagé sur la paroi du tube creux. Cette fenêtre, ou chacune de ces fenêtres (dans l'exemple ci-après on supposera qu'il en existe plusieurs) permet de mettre en regard, sans obstacle galvanique, une région de la surface du microcâble 14 logé dans le tube creux du microcathéter au droit de cette fenêtre, avec un site de stimulation défini sur la paroi de la veine-cible en regard de la fenêtre 16, de manière à constituer ainsi une électrode de détection/stimulation.

L'orifice de la fenêtre 16 peut être vide, c'est-à-dire être laissé tel quel en définissant un volume libre après ablation du matériau du microcathéter à l'endroit des fenêtres 16, ou bien être rempli d'un matériau électriquement conducteur tel qu'un polymère conducteur ou un élément métallique englobé dans la section du microcathéter 12. Mais dans l'un ou l'autre cas, cet orifice est tel qu'il autorise localement la transmission du champ électrique de stimulation et définit de cette manière une zone de stimulation de la microsonde.

En ce qui concerne plus précisément le microcâble 14, celui-ci présente une structure comparable à ce qui est décrit dans le EP 2 581 107 A1 précité. Ce microcâble comprend un câble de coeur plein, électriquement conducteur, possédant une structure composite constituée à partir d'un ou plusieurs torons d'une pluralité de brins élémentaires combinant un matériau structurant et un matériau radio-opaque, avantageusement entre 15 et 300 brins élémentaires. Le matériau structurant peut être notamment un acier inox, un alliage de cobalt, un métal précieux, du titane ou un alliage NiTi présentant une résistance à la fatigue élevée afin de procurer au microcâble les propriétés requises de robustesse, flexibilité, résistance à la fatigue, etc., ainsi qu'une bonne conductibilité électrique. Le matériau radio-opaque, destiné à rendre le microcâble visible aux rayons X pendant sa mise en place par le médecin, peut être choisi parmi le tantale, le tungstène, l'iridium, le platine, l'or et leurs alliages.

Par ailleurs, des moyens de dégressivité de rigidité sont ménagés le long du microcâble entre sa partie proximale et sa partie distale, par exemple par un empilement étagé de tubes emboités les uns dans les autres, ou par une succession de tubes isodiamètre de rigidité croissante. Le gradient de rigidité résultant garantit, d'une part, une partie distale souple non traumatique permettant de suivre toutes les tortuosités du réseau coronaire profond et, d'autre part, une partie proximale plus rigide permettant de transmettre la poussée exercée par le médecin au moyen de dispositifs appropriés.

À l'état libre, le microcathéter peut être rectiligne ou, de préférence, conformé, par exemple au moyen de manchons spécifiques 18 gainant le microcâble dans une région de celui-ci non électriquement active (non située au droit d'une fenêtre 16 du microcathéter 12).

Enfin, à son extrémité proximale le microcâble 14 est pourvu d'un connecteur 20, par exemple de type IS-1, comportant une borne de connexion électrique à un générateur d'un implant cardiaque tel qu'un stimulateur ou resynchroniseur.

À la différence toutefois du microcâble décrit dans le EP 2 581 107 A1 précité, le microcâble 14 de l'invention ne comporte pas de couche isolante, au moins dans sa partie distale située en regard des fenêtres 16 du microcathéter 12.

En effet, comme on le verra plus précisément dans la description détaillée ci-après, c'est le microcathéter 12 qui joue le rôle d'isolant, interposé (sauf localement à l'endroit des fenêtres 16) entre le microcâble 14 et les tissus cardiaques environnants.

Il n'est de ce fait pas nécessaire que le microcâble soit isolé, à tout le moins dans sa partie distale active. L'isolant, par exemple une couche de PET, peut n'être présent que dans la partie proximale, non active, allant jusqu'au connecteur 20, comme illustré en 26 sur la Figure 2.

Le microcathéter 12, quant à lui, est un microcathéter implantable, c'est-à-dire qu'il est apte et destiné à être laissé en place après implantation de la microsonde, ce qui suppose qu'on lui ait conféré des propriétés d'implantabilité que ne possèdent pas les cathéters conventionnels utilisés pour ces interventions (cathéters qui ne servent que le temps de la pose).

Par "implantabilité", on entendra l'aptitude à une implantation permanente dans le réseau coronaire, en toute sécurité pour le patient tant au niveau du matériau choisi - qui doit être biocompatible et notamment hémocompatible - que de l'absence de risques mécaniques, et ceci pendant une durée pouvant atteindre plusieurs années : en particulier, le microcathéter devra pouvoir résister sans rupture typiquement à 400 000 000 de sollicitations en flexion, valeur correspondant au nombre moyen de battements cardiaques sur la durée de vie nominale de la microsonde (10 ans). Autrement dit, ce microcathéter devra pouvoir passer les tests d'implantabilité permanente jusqu'à présent réservés aux sondes, en particulier : biocompatibilité garantie selon ISO 10993 (dispositifs médicaux en général) et EN 45502 (concernant plus particulièrement les sondes) ; absence de corrosion ; absence de risques de rupture ; en cas de rupture, innocuité pour le patient.

Un tel microcathéter implantable est par exemple constitué d'un tube polymère muni à son extrémité proximale d'un système de serrage 22, par exemple par ligature, permettant en fin d'intervention de solidariser axialement au microcathéter le microcâble 14 qui aura été introduit à l'intérieur de la lumière du microcathéter et d'éviter ainsi tout déplacement axial relatif de ces deux éléments.

Le tube du microcathéter 12 présente une section progressivement réduite, par exemple de *Ø₁* = 1,3 mm en partie proximale jusqu'à *Ø₂*= 0,5 à 1,0 mm en partie distale. La structure du microcathéter ne présente en direction longitudinale aucune discontinuité de type soudure ou collage, de manière à minimiser les risques de rupture en fatigue.

Cette configuration procure une très grande souplesse en partie distale et une plus grande rigidité en partie proximale, la transition d'une partie à l'autre étant obtenue de façon continue sans risque de création locale de contraintes excessives lors de la manipulation.

Le tube du microcathéter 12 est avantageusement réalisé par une technique d'extrusion à débit variable de deux matières avec, à l'intérieur, un tube définissant la lumière interne, par exemple en PTFE, matériau choisi pour ses propriétés de très faible coefficient de frottement (facilitant donc l'insertion et le déplacement du microcathéter 14 dans la lumière interne), ses capacités à être extrudé avec un polyuréthanne dans de très faibles épaisseurs, et sa souplesse mécanique. À l'extérieur, le tube en PTFE reçoit par extrusion un revêtement par exemple de polyuréthanne, matériau choisi pour ses propriétés de souplesse, de résistance mécanique, de résistance à l'abrasion, de capacité à être extrudé et de biocompatibilité. Il est possible de prévoir à la surface externe du microcathéter un revêtement biocompatible améliorant le glissement contre les parois des vaisseaux, par exemple un revêtement hydrophile de type polyvinylpyrrolidone (PVP) ou silicone. Le revêtement de surface peut également être choisi pour renforcer la biocompatibilité avec le milieu vivant, par ajout d'agents antimicrobiens et hémocompatibles évitant la formation de thromboses, par exemple un revêtement d'un film mince de carbone.

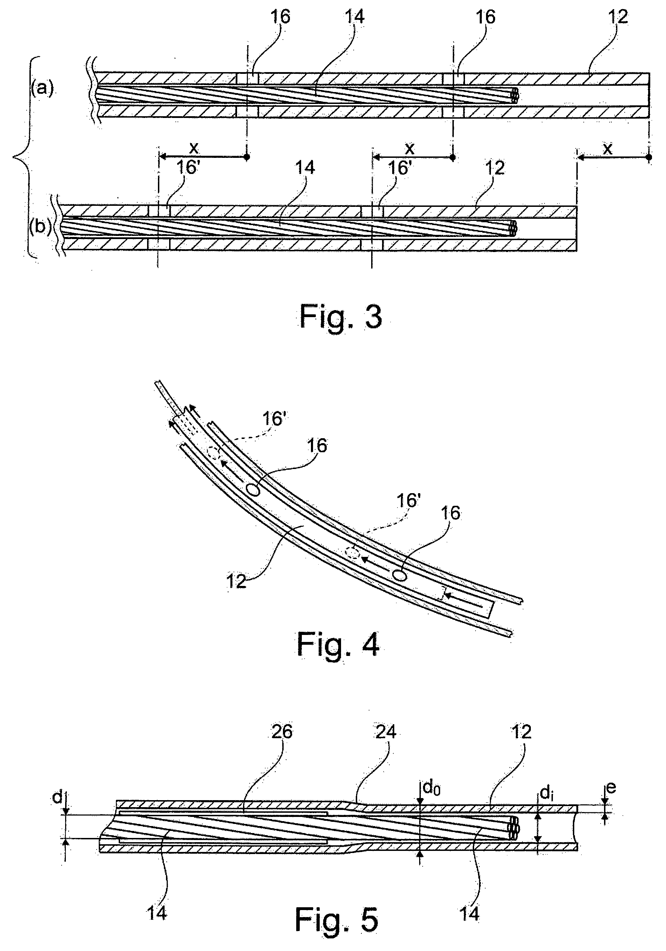

Les Figures 3 et 4 illustrent la possibilité de déplacement du microcathéter sur le microcâble, pour l'ajustement local des zones de stimulation.

On voit que la configuration relative du microcâble 14 et du microcathéter 12 (qui forment ensemble la microsonde de stimulation de l'invention) peut être modifiée, en déplaçant axialement le microcathéter 12 par rapport au microcâble 14, qui reste fixe. Le déplacement, d'amplitude x typiquement comprise entre 0 et 50 mm, permet de modifier, en 16', la position des fenêtres 16, et donc des sites de stimulation, par rapport à la paroi du vaisseau où est implantée la microsonde.

Cette fonction de déplacement télescopique permet en particulier d'ajuster la position des fenêtres de stimulation en fonction des performances électriques relevées au cours de l'implantation, de manière à optimiser les seuils de stimulation, la position des électrodes, d'échapper à la stimulation du nerf phrénique, etc.

La Figure 5 présente une vue en coupe avec les divers diamètres de la combinaison microcathéter 12/microcâble 14, dont les dimensions, bien entendu données à titre indicatif, sont par exemple les suivantes :
- diamètre d du microcâble : 0,3 mm pour le microcâble nu, et 0,34 mm dans une région non active où il est revêtu d'une couche isolante 26 ;
- diamètre intérieur *dᵢ* du microcathéter 12 : 0,35 mm ;
- diamètre extérieur *dₒ* du microcathéter 12 : 0,50 mm ;
- épaisseur du microcathéter : 0,125 mm, réduite à 0,075 mm en partie distale pour conserver un diamètre sensiblement constant.

Un cône 24 permet d'optimiser le passage entre la zone revêtue d'un isolant 26 et celle où le microcâble 14 est à nu, de manière à s'approcher au plus près du conducteur. Il est en effet important de placer la surface conductrice du microcâble au plus près de la paroi veineuse dans la région de la fenêtre du microcathéter, notamment par réduction du jeu entre microsonde et microcathéter (jeu maximal de 0,1 mm sur le diamètre) ainsi que de l'épaisseur du microcathéter dans la partie la plus distale.

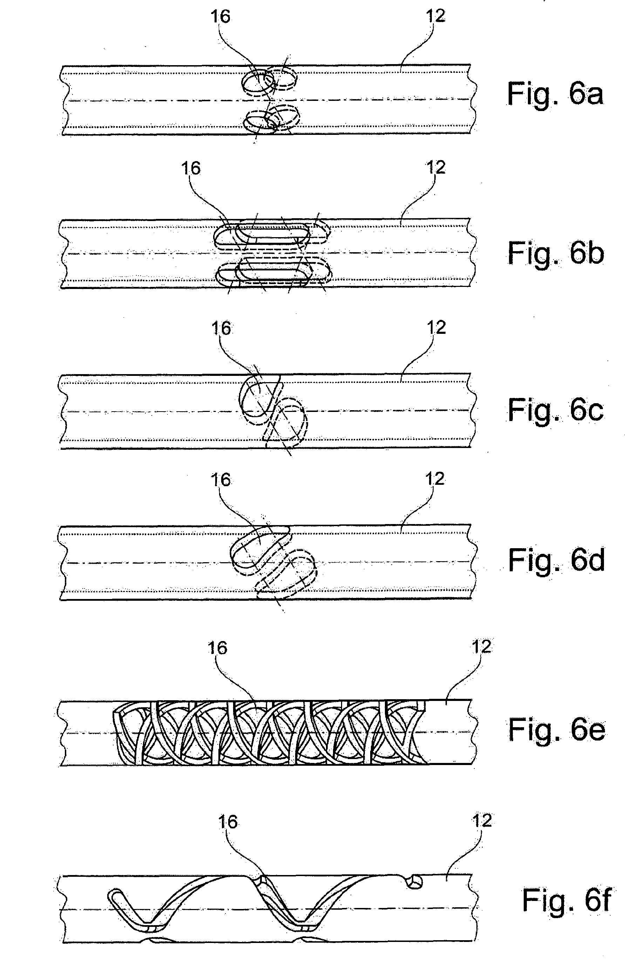

Les Figures 6a à 6f illustrent différentes réalisations possibles des fenêtres ménagées dans le microcathéter de la sonde de l'invention. Ces fenêtres 16 peuvent être notamment formées par :
- quatre trous circulaires répartis circonférentiellement à 90° (Figure 6a) ;
- quatre trous oblongs répartis circonférentiellement à 90° (Figure 6b) ;
- deux trous oblongs répartis circonférentiellement à 90° (Figure 6c) ;
- deux trous oblongs répartis circonférentiellement à 180 ° et présentant une inclinaison de 45° par rapport à une direction circonférentielle (Figure 6d) ;
- des ouvertures multiples réalisées sur une longueur de 10 mm par exemple (Figure 6e) ; ou encore
- deux ouvertures de forme semi-hélicoïdale à 180° (Figure 6f).

Chacune des fenêtres 16 peut être réalisée par exemple par ablation laser sur le tube extrudé. La surface minimale pour permettre une stimulation satisfaisante est de l'ordre de 0,2 mm² par fenêtre, avec de préférence une surface cumulée maximale n'excédant pas 6 mm² pour l'ensemble de la microsonde.

On notera enfin qu'il est possible d'envisager de très nombreuses variantes de la configuration microcathéter/microcâble selon l'invention que l'on vient de décrire.

En particulier, si la configuration décrite correspond à une microsonde monopolaire, avec un conducteur unique délivrant des impulsions simultanément aux divers sites de stimulation, il est également possible d'envisager une configuration multipolaire : dans ce dernier cas, le microcathéter comprend plusieurs conducteurs distincts, isolés entre eux et activables sélectivement par des bornes correspondantes du générateur. Les différents conducteurs du microcâble se présentent alors en vis-à-vis de fenêtres respectives du microcathéter, ces fenêtres correspondant à des sites de stimulation, ou à des groupes de sites de stimulation, activables chacun de manière indépendante.

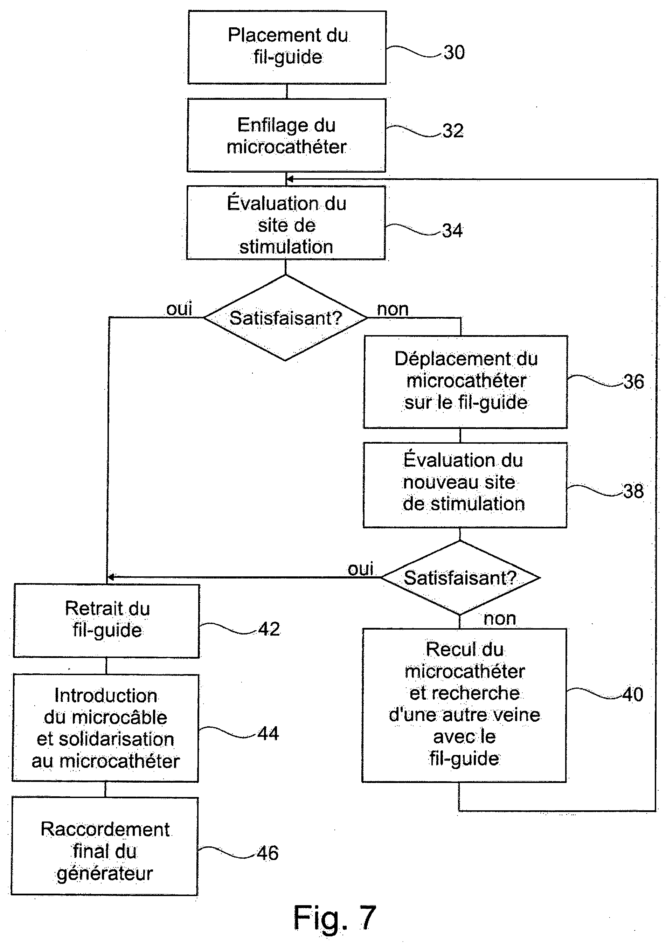

La Figure 7 est un organigramme explicitant les étapes successives de la procédure d'implantation de la microsonde selon l'invention.

L'implantation commence par une mise en place du microcathéter 12, par une technique conventionnelle de type OTW (*Over The Wire*) ou "filoguidage" au moyen d'un mandrin très fin formant fil-guide, pourvu à son extrémité distale d'une terminaison atraumatique permettant son introduction directe dans les vaisseaux du réseau coronaire sans risque de perforation. Au préalable, le médecin dispose un cathéter principal lui permettant d'accéder au débouché du sinus coronaire CS. Un cathéter de sous-sélection est ensuite utilisé pour choisir sous amplificateur de brillance le chemin du réseau coronaire veineux qui permettra d'atteindre la veine-cible.

Le médecin introduit alors le fil-guide dans le cathéter de sous-sélection, et pousse ce fil-guide pour le faire progresser, à nu, dans le réseau coronaire veineux de façon à sélectionner telle ou telle veine collatérale (étape 30), éventuellement jusqu'à une anastomose avec traversée de cette anastomose et remontée dans la veine "retour" choisie.

Le médecin enfile ensuite sur le fil-guide le microcathéter 12 selon l'invention, qu'il fait glisser et progresser sur ce fil-guide jusqu'à l'extrémité de ce dernier (étape 32), en prenant soin que côté distal la totalité de la longueur du fil-guide soit recouverte par le microcathéter.

Dans une procédure conventionnelle, le médecin retirerait ensuite le fil-guide, en laissant en place le microcathéter pour permettre d'y introduire une microsonde.

Tel n'est pas le mode opératoire choisi dans le cas de l'invention.

En effet, dans la mesure où le fil-guide est entièrement recouvert par le microcathéter, il se comporte électriquement de la même manière que le microcâble qui sera enfilé plus tard dans le microcathéter, c'est-à-dire que dans les régions des fenêtres 16 du microcathéter 12, le fil-guide va se trouver en regard de la paroi de la veine-cible sans obstacle, donc avec possibilité de disposer d'une zone de stimulation à cet endroit si le fil-guide est raccordé à un générateur d'impulsions. Pour cela le fil-guide, qui est un fil en matériau conducteur non isolé, est muni à sa partie proximale de moyens de couplage à un tel générateur d'impulsions de stimulation.

Le médecin peut ainsi évaluer l'efficacité du site de stimulation (étape 34) directement avec le fil-guide et à travers les fenêtres du microcathéter.

Si le site n'est pas satisfaisant, le microcathéter est reculé sur le fil-guide pour déplacer la position des fenêtres, de la même manière que ce qui avait été illustré et décrit sur les Figures 3 et 4 plus haut (mais dans ce cas avec le fil-guide introduit dans la lumière du microcathéter en lieu et place du microcâble).

Le nouveau site de stimulation, avec le microcathéter reculé, fait l'objet d'une nouvelle évaluation (étape 38).

Si aucune position du microcathéter dans son déplacement télescopique par rapport au fil-guide ne procure une stimulation satisfaisante, le médecin recule encore plus le microcathéter pour découvrir largement le fil-guide, avec lequel il peut alors sélectionner une autre veine (étape 40). Le microcathéter est alors remis en place de manière à recouvrir complètement le fil-guide, puis la procédure d'évaluation exposée plus haut est réitérée, avec ajustement de la position du site de stimulation par déplacement télescopique du microcathéter sur le fil-guide dans la nouvelle veine sélectionnée (étapes 34, 36, 38).

Lorsqu'un site de stimulation satisfaisant est trouvé, alors le fil-guide est complètement retiré (étape 42), laissant en place le seul microcathéter dans une position inchangée, c'est-à-dire à l'endroit où les fenêtres 16 définissent la position du site de stimulation satisfaisant. Le microcâble 14 est alors introduit dans le microcathéter en remplacement du fil-guide, et solidarisé en position axiale au microcathéter au moyen du système de serrage 22, par exemple par ligature (étape 44).

La microsonde selon l'invention, avec le microcâble 14 et le microcathéter 12 dont les fenêtres 16 ont été judicieusement déplacées au site de stimulation le plus performant, est alors raccordée au générateur du stimulateur ou resynchroniseur cardiaque (étape 46).

## Revendications

1. Un ensemble, implantable dans le réseau veineux coronarien, pour la stimulation d'une cavité cardiaque gauche au moyen d'un générateur de dispositif médical actif implantable, notamment un stimulateur ou resynchroniseur cardiaque, cet ensemble comprenant, en combinaison :
- un microcâble (14) en matériau conducteur, comprenant en partie proximale des moyens (20) de couplage au générateur du dispositif médical actif implantable ; et
- un cathéter (12) en matériau isolant, comprenant un tube creux logeant dans une lumière interne le microcâble avec possibilité de translation axiale relative entre cathéter et microcâble,
ensemble **caractérisé en ce que** :
- le cathéter est un microcathéter implantable de diamètre au plus égal à 2 French (0,66 mm), réalisé en matériau(x) biocompatible(s) adapté(s) à une implantation permanente dans des réseaux veineux ;
- dans sa partie distale, le microcathéter comprend au moins une fenêtre latérale (16) comprenant un orifice traversant ménagé sur la paroi du tube creux, et apte à mettre en regard, sans obstacle galvanique, une région de la surface du microcâble située dans ladite lumière interne au droit de cette fenêtre avec un site de stimulation défini sur la paroi de la veine-cible en regard de la fenêtre du microcathéter, ladite région formant électrode de stimulation ;
- dans sa partie distale, le microcâble est un microcâble non isolé au moins dans la région de la fenêtre du microcathéter ; et
- le microcathéter est apte à être déplacé télescopiquement sur le microcâble dans ladite partie distale entre une position déployée, en rapprochement des extrémités distales respectives du microcâble et du microcathéter, et une position rétractée, en éloignement desdites extrémités distales respectives,
de manière à modifier la position dudit site de stimulation de la veine-cible par l'effet du déplacement du microcathéter sur le microcâble.

2. L'ensemble de la revendication 1, dans lequel l'orifice traversant d'au moins une fenêtre latérale (16) du microcathéter (12) est un orifice vide définissant un volume libre.

3. L'ensemble de la revendication 1, dans lequel l'orifice traversant d'au moins une fenêtre latérale (16) du microcathéter (12) est un orifice rempli d'un matériau électriquement conducteur.

4. L'ensemble de la revendication 1, dans lequel en position déployée le microcâble n'émerge pas au-delà de l'extrémité distale du tube creux du microcathéter.

5. L'ensemble de la revendication 1, dans lequel le microcathéter comprend en outre dans sa région proximale des moyens (22) de solidarisation au microcâble en une position axiale relative, finale, comprise entre la position déployée et la position rétractée.

6. L'ensemble de la revendication 1, dans lequel le microcâble comprend au moins un manchon de conformation (18), gainant le microcâble dans une région de celui-ci située à distance de la fenêtre du microcathéter.

7. L'ensemble de la revendication 1, dans lequel la course de déplacement (x) du microcathéter sur se microcâble entre la position déployée et la position rétractée est d'au plus 50 mm.

8. L'ensemble de la revendication 1, dans lequel la surface unitaire de la au moins une fenêtre latérale est d'au moins 0,2 mm².

9. L'ensemble de la revendication 1, comprenant une pluralité de fenêtres latérales dont la surface cumulée est d'au plus 6 mm².

10. L'ensemble de la revendication 1, dans lequel le microcathéter comprend au moins un groupe d'une pluralité de fenêtres latérales réparties circonférentiellement en une même position axiale du tube creux.

11. L'ensemble de la revendication 1, comprenant en outre :
- un fil-guide amovible non implantable, ce fil-guide étant apte, après avoir été préalablement introduit dans le réseau veineux coronaire jusqu'à une veine-cible, à recevoir le tube creux du microcathéter enfilé dessus, et à permettre par coulissement le guidage du microcathéter jusqu'à la veine-cible,
et dans lequel :
- le fil-guide est en un matériau conducteur non isolé, et il est muni à sa partie proximale de moyens de couplage à un générateur d'impulsions de stimulation ;
- le microcathéter est apte à être déplacé télescopiquement sur le fil-guide entre lesdites positions déployée et rétractée, de manière à définir ledit site de stimulation sur la paroi de la veine-cible en regard de la fenêtre du microcathéter, le fil-guide dans la région de la fenêtre formant électrode de stimulation.

## Patentansprüche

1. Einheit zum Implantieren in den koronaren Venenkreislauf zur Stimulation einer linken Herzhöhle mittels eines Generators einer aktiven implantierbaren medizinischen Vorrichtung, insbesondere eines Herzschrittmachers oder Resynchronisierers, wobei diese Einheit in Kombination Folgendes aufweist:
- ein Mikrokabel (14) aus leitendem Material, das an dem proximalen Teil Mittel (20) zum Koppeln an den Generator der aktiven implantierbaren medizinischen Vorrichtung aufweist; und
- einen Katheter (12) aus isolierendem Material, der einen hohlen Schlauch aufweist, der in einem inneren Lumen das Mikrokabel mit der Möglichkeit der relativen axialen Verschiebung zwischen Katheter und Mikrokabel aufnimmt,
Einheit, die **dadurch gekennzeichnet ist, dass**:
- der Katheter ein implantierbarer Mikrokatheter mit einem Durchmesser von höchstens 2 French (0,66 mm) ist, der aus biokompatiblem Material (biokompatiblen Materialien) hergestellt ist, das (die) für eine dauerhafte Implantation in Venenkreisläufen geeignet ist (sind);
- der Mikrokatheter in seinem distalen Teil mindestens ein seitliches Fenster (16) aufweist, das eine Durchgangsöffnung aufweist, die auf der Wand des hohlen Schlauchs ausgebildet ist, und geeignet ist, einen Bereich der Oberfläche des Mikrokabels, der in dem inneren Lumen unmittelbar an diesem Fenster angeordnet ist, mit einer Stimulationsstelle, die auf der Wand der Zielvene gegenüber von dem Fenster des Mikrokatheters definiert ist, ohne galvanische Barriere gegenüberzustellen, wobei der Bereich eine Stimulationselektrode bildet;
- das Mikrokabel in seinem distalen Teil ein Mikrokabel ist, das mindestens in dem Bereich des Fensters des Mikrokatheters nicht isoliert ist; und
- der Mikrokatheter geeignet ist, auf dem Mikrokabel in dem distalen Teil zwischen einer ausgefahrenen Position durch Annähern der jeweiligen distalen Enden des Mikrokabels und des Mikrokatheters und einer eingefahrenen Position durch Entfernen der jeweiligen distalen Enden derartig teleskopartig verschoben zu werden,
um die Position der Stimulationsstelle der Zielvene durch die Wirkung des Verschiebens des Mikrokatheters auf dem Mikrokabel zu modifizieren.

2. Einheit von Anspruch 1, wobei die Durchgangsöffnung von mindestens einem seitlichen Fenster (16) des Mikrokatheters (12) eine leere Öffnung ist, die ein freies Volumen definiert.

3. Einheit von Anspruch 1, wobei die Durchgangsöffnung von mindestens einem seitlichen Fenster (16) des Mikrokatheters (12) eine Öffnung ist, die mit einem elektrisch leitenden Material gefüllt ist.

4. Einheit von Anspruch 1, wobei das Mikrokabel in der ausgefahrenen Position nicht über das distale Ende des hohlen Schlauchs des Mikrokatheters hinausragt.

5. Einheit nach Anspruch 1, wobei der Mikrokatheter ferner in seinem proximalen Bereich Mittel (22) zum Befestigen an dem Mikrokabel in einer relativen axialen Endposition, die zwischen der ausgefahrenen und der eingefahrenen Position liegt, aufweist.

6. Einheit nach Anspruch 1, wobei das Mikrokabel mindestens eine Formmuffe (18) aufweist, die das Mikrokabel in einem Bereich davon umhüllt, der in Abstand zu dem Fenster des Mikrokatheters angeordnet ist.

7. Einheit von Anspruch 1, wobei der Verschiebungsweg (x) des Mikrokatheters auf dem Mikrokabel zwischen der ausgefahrenen und der eingefahrenen Position höchstens 50 mm beträgt.

8. Einheit von Anspruch 1, wobei die Einheitsfläche des mindestens einen seitlichen Fensters mindestens 0,2 mm² beträgt.

9. Einheit von Anspruch 1, umfassend mehrere seitliche Fenster, deren Gesamtfläche höchstens 6 mm² beträgt.

10. Einheit von Anspruch 1, wobei der Mikrokatheter mindestens eine Gruppe von mehreren seitlichen Fenstern aufweist, die umfangsmäßig in einer gleichen axialen Position des hohlen Schlauchs verteilt sind.

11. Einheit von Anspruch 1, ferner umfassend:
- einen nicht implantierbaren, abnehmbaren Leitdraht, wobei dieser Leitdraht geeignet ist, nachdem er zuvor in den koronaren Venenkreislauf bis zu einer Zielvene eingeführt worden ist, den hohlen Schlauch des Mikrokatheters aufzunehmen, der darüber geschoben ist, und das Führen des Mikrokatheters bis zu der Zielvene durch gleitendes Verschieben zu ermöglichen,
und wobei:
- der Leitdraht aus einem nicht isolierten, leitfähigen Material ist und an seinem proximalen Teil mit Mitteln zum Koppeln an einen Generator von Stimulationsimpulsen ausgestattet ist;
- der Mikrokatheter geeignet ist, auf dem Leitdraht zwischen der ausgefahrenen und der eingefahrenen Position derart teleskopartig verschoben zu werden, um die Stimulationsstelle auf der Wand der Zielvene gegenüber von dem Fenster des Mikrokatheters zu definieren, wobei der Leitdraht in dem Bereich des Fensters eine Stimulationselektrode bildet.

## Claims

1. An assembly, implantable in the coronary venous network, for the stimulation of a left heart chamber by means of a generator of an implantable active medical device, in particular a cardiac stimulator or resynchroniser, said assembly comprising, in combination:
- a micro-cable (14) made of a conductive material, comprising at its proximal part means (20) for its coupling to the generator of the implantable active medical device; and
- a catheter (12) made of an insulating material, comprising a hollow tube housing the micro-cable in an internal lumen with possibility of relative axial translation between the catheter and the micro-cable,
the assembly being **characterized in that**:
- the catheter is an implantable micro-catheter of diameter at most equal to 2 French (0.66 mm), made of biocompatible material(s) adapted for a permanent implantation in venous networks;
- at its distal part, the micro-catheter comprises at least one lateral window (16) comprising a through-orifice formed in the wall of the hollow tube, and adapted to put opposite to each other, with no galvanic obstacle, a region of the micro-cable surface located in said internal lumen in alignment with this window with a stimulation site defined on the wall of the target-vein opposite the micro-catheter window, said region forming a stimulation electrode;
- at its distal part, the micro-cable is a non-insulated micro-cable at least in the region of the micro-catheter window; and
- the micro-catheter is adapted to be telescopically displaced on the micro-cable in said distal part between an extended position, in which the respective distal ends of the micro-cable and the micro-catheter are closer to each other, and a retracted position, in which said respective distal ends are far away from each other,
so as to modify the position of said site of stimulation of the target-vein by displacement of the micro-catheter on the micro-cable.

2. The assembly of claim 1, wherein the through-orifice of at least one lateral window (16) of the micro-catheter (12) is an empty orifice defining a free volume.

3. The assembly of claim 1, wherein the through-orifice of at least one lateral window (16) of the micro-catheter (12) is an orifice filled with an electrically conductive material.

4. The assembly of claim 1, wherein, in the extended position, the micro-cable does not protrude beyond the distal end of the hollow tube of the micro-catheter.

5. The assembly of claim 1, wherein the micro-catheter further comprises in its proximal region means (22) for its fastening to the micro-cable at a final, relative axial position comprised between the extended position and the retracted position.

6. The assembly of claim 1, wherein the micro-cable comprises at least one shaping sleeve (18), cladding the micro-cable in a region of the latter located remote from the micro-catheter window.

7. The assembly of claim 1, wherein the displacement stroke (x) of the micro-catheter on the micro-cable between the extended position and the retracted position is at most of 50 mm.

8. The assembly of claim 1, wherein the unitary surface of the at least one lateral window is of at least 0.2 mm².

9. The assembly of claim 1, comprising a plurality of lateral windows whose cumulated surface is at most of 6 mm².

10. The assembly of claim 1, wherein the micro-catheter comprises at least one group of a plurality of lateral windows circumferentially distributed at a same axial position of the hollow tube.

11. The assembly of claim 1, further comprising:
- a non-implantable removable guide-wire, this guide-wire being adapted, after having been previously introduced in the coronary venous network up to a target-vein, to receive the hollow tube of the micro-catheter threaded thereon, and to allow, by sliding, the guidance of the micro-catheter up to the target-vein,
and in which:
- the guide-wire is made of a non-insulated conductive material, and is provided at its proximal part with means for its coupling to a stimulation pulse generator;
- the micro-catheter is adapted to be telescopically displaced on the guide-wire between said extended and retracted positions, so as to define said stimulation site on the wall of the target-vein opposite the micro-catheter window, the guide-wire in the region of the window forming a stimulation electrode.
